# EUROPÄISCHE PATENTSCHRIFT

(11) **EP 2 670 321 B1**
(45) Veröffentlichungstag und Bekanntmachung des Hinweises auf die Patenterteilung: **10.05.2017**
(21) Anmeldenummer: 12702170.7
(22) Anmeldetag: 30.01.2012
(51) Int. Cl.: A61B 17/70, A61B 17/86

(54) **VORKEHRUNG GEGEN VERKANTEN AN OFFENEN KNOCHENSCHRAUBEN**
PRECAUTION AGAINST JAMMING ON OPEN BONE SCREWS
MESURE PRISE POUR PALLIER LES RISQUES DE DÉVERS INDUISANT DES BLOCAGES AU NIVEAU DE VIS D'OSTÉOSYNTHÈSE OUVERTES

(30) Priorität: 04.02.2011 CH 214112011
(43) Veröffentlichungstag der Anmeldung: 11.12.2013
(73) Patentinhaber: Spinesave AG, 8424 Embrach (CH)
(72) Erfinder: FREUDIGER, Stefan, CH-3047 Bremgarten (CH); DIENER, Rolf, CH-8185 Winkel (CH)
(74) Vertreter: AMMANN PATENTANWÄLTE AG BERN
(86) Internationale Anmeldenummer: PCT/CH2012/000025
(87) Internationale Veröffentlichungsnummer: WO 2012/103660

(56) Entgegenhaltungen:
- EP-A1- 1 426 016
- EP-A1- 1 759 646
- EP-A1- 1 891 904
- WO-A1-03/015648
- DE-A1- 3 916 198
- US-A- 4 929 136
- US-A- 5 320 467

## Beschreibung

Die vorliegende Erfindung bezieht sich auf eine Knochenschraube gemäss Oberbegriff des Anspruchs 1. Insbesondere bezieht sie sich auf die Gewindegeometrie für Befestigungselemente von Verbindungsstäben auf Knochenschrauben mit offenen Aufnahmen.

Mit der vorliegenden Erfindung werden Vorkehrungen offenbart, mit deren Hilfe das Verkanten von aufzusetzenden Innenschrauben oder Aussenmuttern auf offene Knochenschrauben verhindert oder zumindest die Wahrscheinlichkeit des Eintretens eines Verkantens verringert werden kann. Offene Knochenschrauben, insbesondere Pedikelschrauben, haben den grossen Vorteil, dass zum Beispiel wirbelkörperverbindende Stäbe von oben eingelegt werden können. Ein Nachteil hierbei besteht jedoch darin, dass das Gewinde zur Aufnahme eines Befestigungselementes aus zwei Hälften besteht und damit die Gefahr des Verkantens erhöht.

Im Stand der Technik, namentlich mit dem deutschen Stichwort "Verkanten" oder den englischen Stichwörter "jam" respektive "cant" wurden keine vorbekannten Vorkehrungen gegen Verkanten bei offenen Knochenschrauben gefunden. Ein Grund hierfür könnte darin liegen, dass Verbindungsstäbe früher eher aus Metall für Fusionen und heute eher aus Kunststoffen für dynamische Systeme angeboten werden. Metallische Verbindungsstäbe können aufgrund ihrer höheren Festigkeit in geringeren Durchmessern angeboten werden, weshalb die offenen Gewindehälften dicker und umfangsmässig mit einem grösseren Gewindeanteil versehen werden können. Verbindungsstäbe aus Kunststoff hingegen weisen höhere Durchmesser auf, so dass aus Platzgründen die offenen Gewindehälften kleinstmöglich gehalten werden müssen, was zu geringeren Gewindeumschlingungswinkeln führt und damit die Gefahr von Verkanten erhöht.

DE 39 16 198 A1 weist ebenfalls eine Aussenmutter auf, welche auf zwei Aussengewindehälften ansetzt. Hierbei weist die Aussenmutter innseitig eine ringförmige Ausnehmung auf, welche über die Aussengewindehälften gestülpt wird. Die Aussengewinde sind dabei deutlich zurückgenommen, so dass eine Schulter entsteht, welche der Zentrierung dienen kann. Schultern sind bekannte Zentrierhilfen, welche jedoch wesentlich mehr Platz benötigen als die vorliegende Erfindung.

EP 1 759 646 A1 weist ebenfalls eine Aussenmutter mit Füllstück auf, wobei die Verschraubung keine Merkmale gegen Verkanten aufweist.

Die im Folgenden beschriebene Erfindung stellt sich demnach die Aufgabe, Vorkehrungen anzubieten, mit deren Hilfe ein Verkanten einer Innenschraube oder Aussenmutter beim Aufsetzen auf eine offene Knochenschraube verhindert werden soll. Dabei hat die Innenschraube oder die Aussenmutter im Sinne eines Befestigungselementes die Aufgabe, einen durch die offene Knochenschraube eingelegten Verbindungsstab in der Knochenschraube zu befestigen.

Eine derartige Knochenschraube ist im Anspruch 1 angegeben. Die weiteren Ansprüche geben bevorzugte Ausführungen an.

Die Lösung dieser Aufgabe zeichnet sich dadurch aus, dass die einlaufenden Gewindegänge keine angeschnittenen Segmente aufweisen, welche aufgrund ihrer Anschrägung ein Verkanten provozieren könnten.

Der Gegenstand der Erfindung ist folglich die im Patentanspruch 1 definierte spezielle Gewindeeinlaufgeometrie.

Die vorliegende Erfindung wird nachfolgend anhand von bevorzugten Ausführungsbeispielen unter Bezugnahme auf die Zeichnungen näher erläutert.

Es zeigen schematisch:
- Fig. 1: eine Knochenschraube mit offener Aufnahme für einen Verbindungsstab.
- Fig. 2a: eine Stabbefestigung mit Aussenmutter und Füllstück.
- Fig. 2b: Stabbefestigung mit einteiliger Aussenmutter und integriertem Füllstück.
- Fig. 3a: eine Stabbefestigung mit Innenschraube und Füllstück.
- Fig. 3b: eine Stabbefestigung mit einteiliger Innenschraube und integriertem Füllstück.
- Fig. 4: eine Knochenschraube mit nur ganzen Gewindegängen auf der Aussenseite der Stab-Aufnahme (Vaterstück an Knochenschraube).
- Fig. 5a: eine Aussenmutter im Schnitt mit nur ganzen Gewindegängen auf der Innenseite (Mutterstück im Befestigungselement).
- Fig. 5b: eine Innensicht in die Aussenmutter mit gerundetem Übergang zum ersten Gewindegang in voller Höhe.

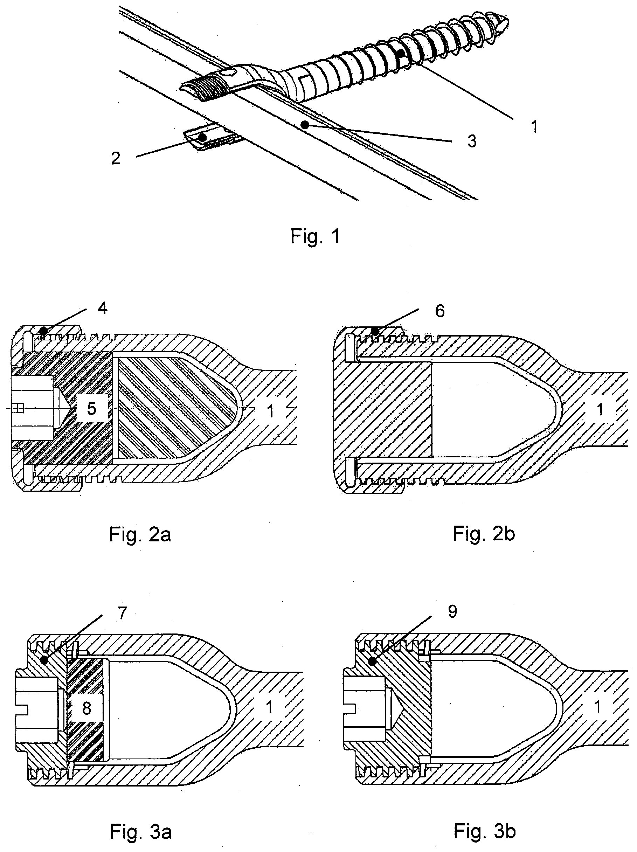

In Fig. 1 wird eine Knochenschraube 1 mit einer offenen Aufnahme bestehend aus je einer Gewindehälfte 2 zur Aufnahme eines Verbindungsstabes 3 gezeigt.

Der Umschlingungswinkel, d.h. der Anteil vom Umfang des Kopfs einer Knochenschraube, der von einer Gewindehälfte gebildet wird, beträgt in der Regel höchstens 150° und oft höchstens 120°. Insbesondere bei Aufnahmen für einen Kunststoffverbindungsstab treten bevorzugt Winkel von 90° und kleiner wegen der relativ zum Kopfquerschnitt grösseren Öffnung auf.

In Fig. 2a wird eine Knochenschraube 1 mit einer Stabbefestigung bestehend aus einer Aussenmutter 4 und Füllstück 5 gezeigt.

In Fig. 2b wird eine Knochenschraube 1 mit einer Stabbefestigung bestehend aus einer Aussenmutter mit integriertem Füllstück 6 gezeigt.

In Fig. 3a wird eine Knochenschraube 1 mit einer Stabbefestigung bestehend aus einer Innenschraube 7 und Füllstück 8 gezeigt.

In Fig. 3b wird eine Knochenschraube 1 mit einer Stabbefestigung bestehend aus einer Innenschraube und integriertem Füllstück 9 gezeigt.

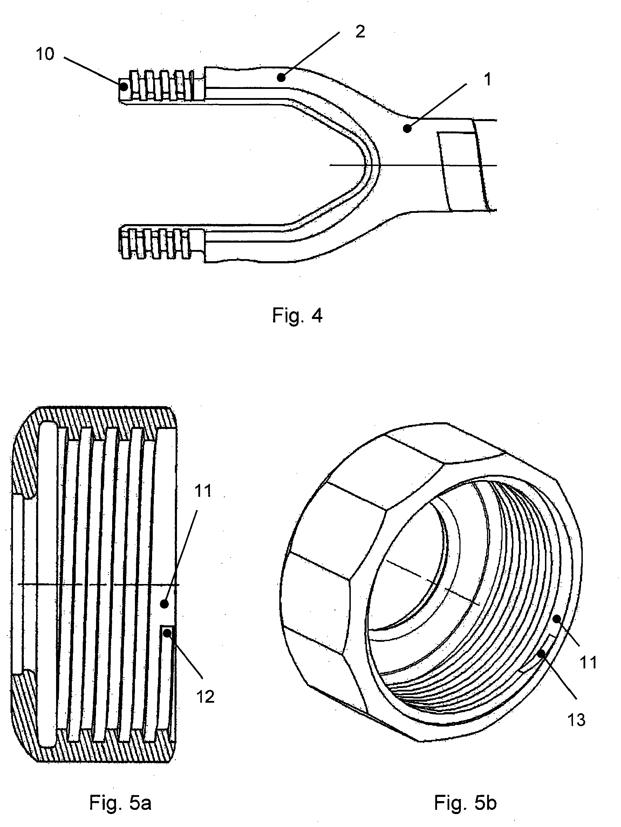

In Fig. 4 wird eine Knochenschraube 1 mit je einer Gewindehälfte 2 mit nur ganzen Gewindegängen 10 auf der Aussenseite der Stab-Aufnahme (Vaterstück an Knochenschraube) gezeigt.

In Fig. 5a wird ein Schnitt einer Aussenmutter mit nur ganzen Gewindegängen 11 (Mutterstück im Befestigungselement) gezeigt, bei welcher der Einlauf des ersten Gewindegangs mit voller Höhe stumpf ist 12.

In Fig. 5b wird eine Innensicht in eine Aussenmutter mit keinen angeschnittenen Gewindegängen 11 (Mutterstück im Befestigungselement) gezeigt, bei welcher der Einlauf des ersten Gewindegangs mit voller Höhe leicht gerundet ist 13.

Ein stumpfer 12 oder nur leicht angerundeter 13 Gewindeeinlauf mit einer Gewindeflanke in voller Höhe hat zusätzlich den Vorteil, dass, wenn er das korrekte Eingreifen nicht auf Anhieb sicherstellen kann, er zu einem raschen und deutlich spürbaren Anschlag führt. In einem solchen Fall genügt es, wenn der Chirurg das Befestigungselement wenig mehr als 180° zurückdreht, was ihn dann automatisch zu der korrekten Relativlage der zusammenzuführenden Gewinde bringt.

Selbstverständlich ist diese Erfindung nicht auf eingängige Gewinde beschränkt, sondern kann sinngemäss auch auf mehrgängige Gewinde angewandt werden.

Die Vorkehrung gegen Verkanten kann weiter unterstützt werden, indem im Falle der Verwendung eines Füllstückes 5, 8 dieses mit engen Toleranzen versehen wird und zur korrekten Zentrierung der Gewindepaare beiträgt.

Die Vorkehrung gegen Verkanten kann weiter unterstützt werden, indem die korrekt passenden Gewindehälften vater- und mutterseitig mit je einer unterschiedlichen Farbe oder einer unterschiedlichen Schraffur markiert werden.
Die Zeichnungen zeigen für das Befestigungselement im Detail ein Vatergewinde an der Aussenseite der Knochenschrauben-Gewindehälften und einem Muttergewinde auf der Innenseite des Befestigungselementes. Selbstverständlich lässt sich diese Erfindung auch umgekehrt anwenden, nämlich auf ein Vatergewinde auf der Aussenseite des Befestigungselementes und ein Muttergewinde an der Innenseite der Knochenschrauben-Gewindehälften, welche hier nicht gezeichnet sind.

Die vorangehende Beschreibung von bevorzugten Ausführungsbeispielen beschränkt nicht den Schutzumfang vorliegender Erfindung, der allein durch die Ansprüche definiert ist, und ermöglicht dem Fachmann Abänderungen und Ergänzungen im Rahmen des Schutzumfangs.

## Patentansprüche

1. Knochenschraube (1) mit zwei Gewindehälften (2) die eine offene Aufnahme für ein Verbindungselement begrenzen, und Befestigungselement (4, 6, 7, 9), wobei die Gewindehälften zur Aufnahme des Befestigungselements ausgebildet sind, **dadurch gekennzeichnet, dass** mindestens ein Befestigungsglied ausgewählt aus den Gewindehälften und dem Befestigungselement einen Gewindeeinlauf aufweist, bei dem Gewindegänge erst dann vorhanden sind, wenn diese in voller Flankenhöhe ausgebildet sind.

2. Knochenschraube (1) und Befestigungselement (4, 6, 7, 9) nach Anspruch 1, **dadurch gekennzeichnet, dass** das Befestigungselement eine Aussenmutter (4, 6) ist und sich ein Gewinde zu dessen Aufnahme auf der Aussenseite der Knochenschrauben-Gewindehälften (2) befindet.

3. Knochenschraube (1) und Befestigungselement (4, 6, 7, 9) nach Anspruch 1, **dadurch gekennzeichnet, dass** das Befestigungselement eine Innenschraube (7, 9) ist und sich ein Gewinde zu dessen Aufnahme auf der Innenseite der Knochenschrauben-Gewindehälften (2) befindet.

4. Knochenschraube **(1)** und Befestigungselement (4, 6, 7, 9) nach Anspruch **2** oder **3**, **dadurch gekennzeichnet, dass** das Gewinde einen oder mehrere Gänge hat.

5. Knochenschraube (1) und Befestigungselement (4, 6, 7, 9) nach Anspruch **4**, **dadurch gekennzeichnet, dass** das Befestigungselement mit einem Füllstück (5, 6, 8, 9) kombiniert wird, welches zusätzlich als Zentrierhilfe dient.

6. Knochenschraube (1) und Befestigungselement (4, 6, 7, 9) nach Anspruch **4**, **dadurch gekennzeichnet, dass** ein zusätzlicher Verkantschutz in der Form einer unterschiedlichen Farbmarkierung oder Schraffur je Gewindehälfte und zugehöriger Hälfte des Befestigungselementes angebracht ist.

7. Knochenschraube (1) und Befestigungselement (4, 6, 7, 9) gemäss einem der Ansprüche 1 bis 6, **dadurch gekennzeichnet, dass** an dem Befestigungselement und **an** den Gewindehälften (2) Gewindegänge erst dann vorhanden sind, wenn diese in voller Flankenhöhe ausgebildet sind.

## Claims

1. Bone screw (1) having two thread halves (2) delimiting an open seat for a connecting element, and fastening element (4, 6, 7, 9), the thread halves being designed for receiving the fastening element, **characterised in that** at least one fastening member selected from the threads halves and the fastening element has a thread lead-in where threads are only present from the point where their flanks have the full height.

2. Bone screw (1) and fastening element (4, 6, 7, 9) according to claim 1, **characterised in that** the fastening element is a screw nut (4, 6) and a thread for receiving the latter is situated on the exterior of the bone screw thread halves (2).

3. Bone screw (1) and fastening element (4, 6, 7, 9) according to claim 1, **characterised in that** the fastening element is a screw (7, 9) and a thread for receiving the latter is situated on the inside of the bone screw thread halves (2).

4. Bone screw (1) and fastening element (4, 6, 7, 9) according to claim 2 or 3, **characterised in that** the thread has one or multiple turns.

5. Bone screw (1) and fastening element (4, 6, 7, 9) according to claim 4, **characterised in that** the fastening element is combined with a spacer (5, 6, 8, 9) which additionally serves as a centring aid.

6. Bone screw (1) and fastening element (4, 6, 7, 9) according to claim 4, **characterised in that** an additional canting protection in the form of different colour marks or hatched areas on each thread half and the associated half of the fastening element is provided.

7. Bone screw (1) and fastening element (4, 6, 7, 9) according to one of claims 1 to 6, **characterised in that** on the fastening element and on the thread halves (2), threads are only present from the point where where their flanks have the full height.

## Revendications

1. Vis à os (1) ayant deux moitiés de filetage (2) délimitant un emplacement destiné à recevoir un élément de liaison, et élément de fixation (4, 6, 7, 9), les moitiés de filetage étant façonnées de manière à recevoir ledit élément de fixation, **caractérisés en ce qu'**au moins un organe de fixation choisi parmi les moitiés de filetage et l'élément de fixation présente une entrée de filetage où des filets ne sont pourvus qu'à partir du point où leurs flancs ont la pleine hauteur.

2. Vis à os (1) et élément de fixation (4, 6, 7, 9) selon la revendication 1, **caractérisés en ce que** l'élément de fixation est un écrou (4, 6) et qu'un filetage qui le reçoit se trouve sur l'extérieur des moitiés de filetage (2) de la vis à os.

3. Vis à os (1) et élément de fixation (4, 6, 7, 9) selon la revendication 1, **caractérisés en ce que** l'élément de fixation est une vis (7, 9) et qu'un filetage qui la reçoit se trouve à l'intérieur des moitiés de filetage (2) de la vis à os.

4. Vis à os (1) et élément de fixation (4, 6, 7, 9) selon la revendication 2 ou 3, **caractérisés en ce que** le filetage comprend un ou plusieurs filets.

5. Vis à os (1) et élément de fixation (4, 6, 7, 9) selon la revendication 4, **caractérisés en ce que** l'élément de fixation est combiné à une pièce intercalaire (5, 6, 8, 9) qui en plus sert d'aide de centrage.

6. Vis à os (1) et élément de fixation (4, 6, 7, 9) selon la revendication 4, **caractérisés en ce qu'**une protection supplémentaire contre le blocage est pourvue sous la forme de marques de couleur ou zones hachurées différentes sur chaque moitié filetée et moitié correspondante de l'élément de fixation.

7. Vis à os (1) et élément de fixation (4, 6, 7, 9) selon l'une des revendications 1 à 6, **caractérisés en ce que** des filets ne sont pourvus qu'à partir du point où leurs flancs ont la pleine hauteur sur l'élément de fixation et sur les moitiés de filetage (2).
